# EUROPÄISCHE PATENTSCHRIFT

(11) **EP 0 645 990 B1**
(45) Veröffentlichungstag und Bekanntmachung des Hinweises auf die Patenterteilung: **01.09.1999**
(21) Anmeldenummer: 93912977.1
(22) Anmeldetag: 15.06.1993
(51) Int. Cl.: A61F 2/46

(54) **APPLIKATIONSSYSTEM**
APPLICATOR SYSTEM
DISPOSITIF D'APPLICATION

(30) Priorität: 15.06.1992 DE 4219563
(43) Veröffentlichungstag der Anmeldung: 05.04.1995
(73) Patentinhaber: Draenert, Klaus, Dr.med.Dr.med.habil., D-81545 München (DE)
(72) Erfinder: Draenert, Klaus, Dr.med.Dr.med.habil., D-81545 München (DE)
(86) Internationale Anmeldenummer: EP9301510
(87) Internationale Veröffentlichungsnummer: WO9325162

(56) Entgegenhaltungen:
- EP-A- 0 006 430
- EP-A- 0 108 584
- CH-A- 639 549
- DE-A- 4 022 985
- DE-A- 4 022 986
- FR-A- 2 656 521
- US-A- 4 338 925
- US-A- 4 625 722

## Beschreibung

Die Erfindung betrifft ein mehrteiliges Applikationssystem für plastische Massen, beispielsweise plastische organische Kunststoffe, wie PMMA-Knochenzemente, oder anorganische Zementmassen, insbesondere zum Applizieren von Knochenzement in eine Markhöhle, wie es im Oberbegriff des Anspruchs 1 definiert wird. Ein derartiges Applikationssystem ist z.B. aus der US-A-4 625722 bekannt.

Wie beispielsweise in der EP-A-170 120 und der EP-A-262 185 beschrieben, wird Knochenzement meist aus einem vorzugsweise zylindrischen Behältnis mit einer vorzugsweise queren Auslaßöffnung heraus mittels einer Pistole oder Spritze in die Markhöhle appliziert. Dabei sollte dafür gesorgt werden, daS der Knochenzement möglichst ohne Verluste in die Markhöhle eingebracht werden kann und die Markhöhle ganz ausfüllt. Ähnliche Vorgaben sind auch bei der Applikation anderer plastischer Massen in zu befüllende Räume zu erfüllen. Dies ist z.B. dann besonders wichtig, wenn aufgrund genauer Dosierungsvorschriften sichergestellt sein muß, daß die zuvor abgemessene plastische Masse vollständig in den zu befüllenden Raum überführt wird.

Der Erfindung liegt somit die Aufgabe Zugrunde, eine Vorrichtung bereitzustellen, mit der eine plastische Masse aus einem Behältnis heraus ohne Verluste in einen zu befüllenden Raum eingebracht werden kann und den Zu befüllenden Raum möglichst vollständig ausfüllt.

Diese Aufgabe wird durch das Applikationssystem gemäß Anspruch 1 gelöst.

Die Erfindung geht dabei von dem Grundgedanken aus, ein Applikationssystem mit einem mit dem Behältnis verbindbaren und vorzugsweise einen trichterförmigen Teil aufweisenden Auslaßstück und einem länglichen Applikationsteil vorzusehen, wobei entlang der inneren Oberflächen die Übergänge zwischen dem Behältnis und dem Auslaßstück sowie dem Auslaßstück und dem Applikationsteil jeweils bündig bzw. glatt verlaufen und kein Absatz vorhanden ist, der die Strömung der plastischen Masse beeinträchtigen könnte.

Das erfindungsgemäße Applikationssystem kann mehrteilig, insbesondere zweiteilig ausgebildet sein. Beispielsweise kann das Applikationssystem als ein- oder mehrteilige Kappe ausgebildet sein, die auf das Behältnis, beispielsweise eine Knochenzementkartusche, aufsetzbar ist. Die Kappe bzw. das Auslaßstück und das Applikationsteil können entweder einstückig oder als mehrere miteinander verbindbare Teile ausgebildet sein.

Wenn das Behältnis für die plastische Masse zylindrisch ist, ist das Auslaßstück des Applikationssystems vorzugsweise trichterförmig ohne Absatz zum Behältnis entlang deren innerer Oberflächen ausgebildet. An das trichterförmige Auslaßstück schließt sich ohne Absatz entlang der inneren Oberflächen das vorzugsweise etwa 3 bis 25 cm, besonders bevorzugt etwa 5 bis 20 cm lange Applikationsteil an, das als Schlauch oder Schnorchel ausgebildet sein kann.

Besonders bevorzugt ist das Applikationssystem als ein- oder mehrteilige Kappe ausgeführt, die derart auf die Auslaßöffnung des Behältnisses aufsetzbar ist, beispielsweise mittels eines Bajonettverschlusses oder eines Schraubverschlusses, daß die inneren Oberflächen von Behältnis und Kappe absatzlos ineinander übergehen. Dabei ist die Einstellung der Kappe in ihrer relativen Position, beispielsweise Drehstellung, gegenüber dem Behältnis vorzugsweise reproduzierbar ausgebildet, beispielsweise durch einen Anschlag des Bajonetts oder eine exakte Abstimmung des Gewindes des Schraubenverschlusses, so daß die Kappe jeweils in derselben Position auf das Behältnis aufgesetzt wird. Dies ist besonders dann vorteilhaft, wenn die Auslaßöffnung des Behältnisses nicht rotationssymmetrisch ausgebildet ist und die Auslaßöffnung beispielsweise quer oder länglich ausgebildet ist. In diesem Fall muß auch der Innenumfang der Kappe am Übergang zum Behältnis entsprechend ausgebildet sein und einen länglichen Querschnitt aufweisen. Durch die reproduzierbare Positionierung ist auch bei einer derartigen nicht-rotationssymmetrischen Ausbildung gewährleistet, daß am Übergang kein Absatz entlang der inneren Oberflächen von Behältnis und Kappe entsteht.

Das Applikationssystem ist vorzugsweise zweiteilig ausgebildet und weist neben einem mit dem Behältnis verbindbaren Hauptteil(Kappe) einen aufgesetzten Schlauch auf, der in der Weise aufgezogen ist, daß der innere Durchmesser des Schlauchs im ungedehnten Zustand mit dem inneren Durchmesser der Auslaßöffnung der Kappe identisch ist. Der Schlauch kann vorzugsweise aus Teflon (ein eingetragenes Warenzeichen) bestehen. Der auf den Hauptteil bzw. die Kappe aufgezogene Schlauch wird vorzugsweise mittels mindestens eines scharfkantigen Ringprofils mit Hinterschneidung am Hauptteil bzw. dem Auslaßstück der Kappe festgehalten und am Zurückgleiten gehindert, kann jedoch auch mittels eines Gewindes oder einer Vorrichtung für die Aufnahme einer Schlauchklemme oder durch andere Befestigungseinrichtungen an dem Hauptteil bzw. der Kappe befestigt und gegen ein Abrutschen gesichert sein.

Das längliche Applikationsteil ist faltbar ausgebildet, beispielsweise als sternförmig oder in anderer Weise faltbarer Schlauch oder Schnorchel. Ein derartiger Schlauch oder Schnorchel läßt sich leicht in gefaltetem Zustand in den zu befüllenden Raum, beispielsweise die Markhöhle einführen, und entfaltet sich dann durch die in ihn eingebrachte plastische Masse während der Applikation. Am Ende der Applikation füllt der mit der plastischen Masse gefüllte Schnorchel oder Schlauch den Zu befüllenden Raum ganz aus und kann anschließend zurückgezogen und aus dem zu befüllenden Raum entfernt werden, wobei die plastische Masse zurückbleibt. Das Einführen des faltbaren Applikationsteils in den zu befüllenden Raum wird erleichtert, wenn der Applikationsteil an seinem freien Ende leichter entfaltbar ist als an seinem anderen, mit dem Auslaßstück bzw. dem Kappenhauptteil verbundenen Ende.

Durch das längliche Applikationsteil kann beispielsweise Knochenzement beim Implantieren einer Hüftgelenk-Endoprothese distal in das knöcherne Lager appliziert werden.

Als Material für das Applikationssystem wird vorzugsweise ein sterilisierbarer Kunststoff, beispielsweise TPX, ein Gummi oder Silikon, Polycarbonat oder ein Material mit ähnlichen Eigenschaften, ein Metall oder Glas verwendet. Für den Schnorchel wird besonders bevorzugt ein glattes elastisches Material oder ein Verbundwerkstoff verwendet, welcher Windkesselfunktion entfalten kann, d.h. sich durch die zu applizierende Masse plastisch deformiert und aufbläht und beim anschließenden Zusammenziehen die Masse im Schnorchel vorwärtsbewegt und vorantreibt.

Die Erfindung wird nachstehend anhand der Zeichnung näher erläutert. Es zeigen:
- Fig. 1: eine Draufsicht auf das erfindungsgemäße Applikationssystem aus der Richtung des Behältnisses;
- Fig. 2: eine Seitenansicht des Applikationssystems gemäß Fig. 1 im Schnitt, und
- Fig. 3a und 3b: eine weitere Ausführungsform des erfindungsgemäßen Applikationssystems.

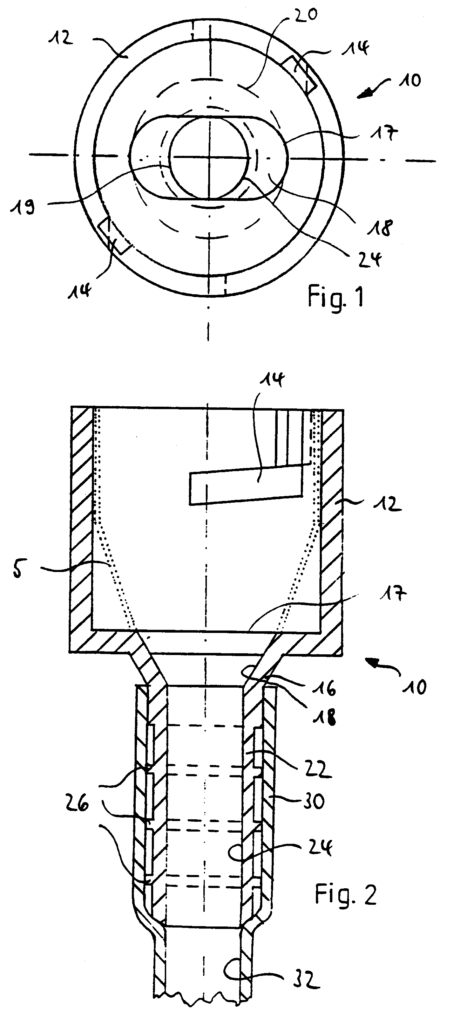

Das Applikationssystem gemäß Figuren 1 und 2 weist eine Kappe 10 auf, deren zylinderförmiges Oberteil 12 an das Behältnis, aus dem heraus die plastischen Stoffe zu applizieren sind, angepaßt ist und den vorderen Teil des Behältnisses bis zu dessen Auslaßöffnung in sich aufnimmt. Das am Außenumfang einen oder mehrere Zapfen aufweisende Behältnis ist mittels eines Bajonettverschlusses 14, in den die Zapfen eingreifen, mit der Kappe 10 verbindbar, wobei die relative Radialstellung von Behältnis und Kappe reproduzierbar einstellbar ist. Die Wand des eingeführten Behältnisses 5 ist in Fig. 2 punktiert eingezeichnet. Mit dem Bezugszeichen 6 ist die Achse von Behältnis und Applikationssystem bezeichnet.

Die Kappe des Applikationssystems weist ferner ein Auslaßstück 16 mit einem trichterförmigen Teil auf. Die obere Mündung 17 des trichterförmigen Teils ist quer bzw. länglich und entspricht in ihrer Form der Auslaßoffnung des Behältnisses. Im montierten Zustand liegt die Mündung 17 im wesentlichen in derselben Ebene wie die Auslaßöffnung des Behältnisses. Die innere Oberfläche 18 des Trichterteils verjüngt sich von der Mündung 17 nach unten derart, daß der untere Querschnitt 19 des Trichters rotationssymmetrisch bzw. rund ist. Wie durch die gestrichelte Linie 20 angedeutet, ist die Außenoberfläche des Trichterteils rotationssymmetrisch.

Der trichterförmige Teil des Auslaßstückes 16 der Kappe 10 geht in einen zylindrischen Teil 22 über, dessen Innenquerschnitt 24 bzw. dessen innere Oberfläche dem Querschnitt 19 entspricht. Der Übergang zwischen trichterförmigem Teil und zylindrischem Teil 22 kann aus strömungstechnischen Gründen abgerundet sein. Am Außenumfang des zylindrischen Teils 22 sind drei Ringprofile 26 mit Hinterschneidungen vorgesehen. Über den zylindrischen Teil 22 ist ein Schlauch 30 aufgezogen, der vorzugsweise im erwärmten Zustand über den zylindrischen Teil 22 geschoben und durch die hinterschnittenen Ringprofile 26 festgehalten wird. Der Schlauch 30 dient als längliches Applikationsteil. Der Innenquerschnitt 32 bzw. die innere Oberfläche des ungedehnten Schlauchs entspricht dem Innenquerschnitt 24 bzw. der inneren Oberfläche des zylindrischen Teils 22 und setzt diese absatzfrei fort. Der Schlauch kann z.B. einen Innendurchmesser von 6 bis 10 mm und eine Wandstärke von 1 mm aufweisen.

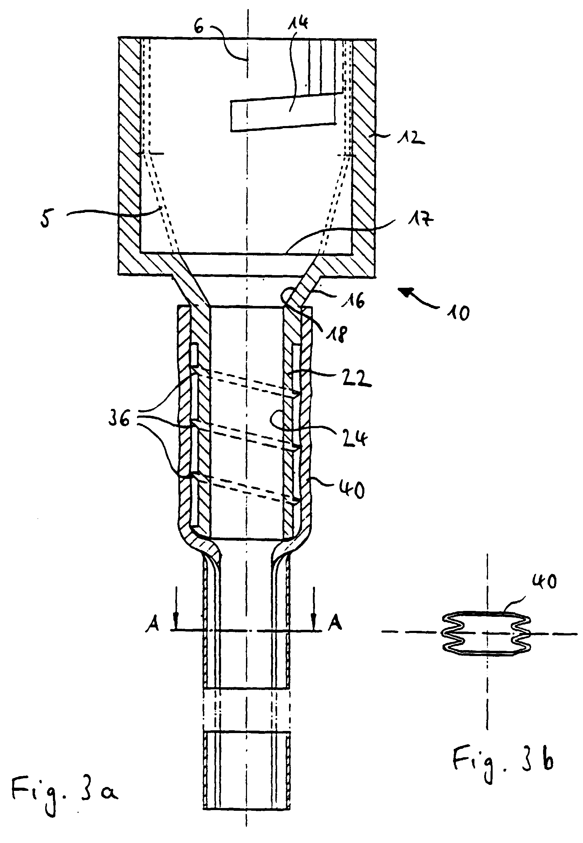

Bei der Ausführungsform gemäß Fig. 3 bezeichnen gleiche Bezugszeichen gleiche Teile wie in den Figuren 1 und 2. Bei der Ausführungsform gemäß Fig. 3 ist am Außenumfang des zylindrischen Teils 22 der Kappe 10 ein Gewinde 36 vorgesehen, durch welches das Applikationsteil an der Kappe (Auslaßstück) befestigt und gesichert ist. Das Gewinde 36 erleichtert das Aufziehen des Applikationsteils. Das Applikationsteil ist in der Ausführungsform gemäß Figuren 3a und 3b als faltbarer Schlauch 40 ausgebildet. Im unteren Teil von Fig. 3a ist die Faltung des Schlauches 40 schematisch dargestellt. Fig. 3b stellt einen Querschnitt entlang der Linie A-A von Fig. 3a dar, in dem die Faltung des Schlauches 40 dargestellt ist. Beim Applizieren der plastischen Masse entfaltet sich der Schlauch 40 derart, daß am Übergang zwischen dem Applikationsteil bzw. der Kappe 10 und dem Schlauch 40 kein Absatz vorhanden ist, und die plastische Masse somit ungehindert strömen kann.

Durch die vorstehend beschriebene Ausgestaltung gemäß Fig. 2 und 3 ist gewährleistet, daß sowohl am Übergang zwischen der Auslaßöffnung des Behältnisses und der Mündung des Trichterteils der Kappe als auch zwischen dem Austrittsende des zylindrischen Teils der Kappe und der inneren Oberfläche des Schlauchs kein Absatz auftritt und dadurch eine problemlose, verlustfreie und vollständige Applikation der plastischen Masse gewährleistet ist.

## Patentansprüche

1. Applikationssystem zur Applikation von Knochenzement aus einem Behältnis (5) in eine Markhöhle, mit einem an das Behältnis angepaßten Auslaßstück (16) und einem länglichen Applikationsteil, dadurch gekennzeichnet, daß entlang der inneren Oberfläche der Teile des Applikationssystems kein Absatz vorhanden ist, und daß das Applikationsteil ein einstückiger faltbarer Schlauch oder Schnorchel (40) ist, der außen am Auslaßstück (16) befestigt ist und dessen Länge und Material so ausgewählt sind, daß der Schlauch oder Schnorchel (40) sich durch den in ihn eingebrachten Knochenzement während der Applikation deformiert und aufbläht, und beim anschließenden Zusammenziehen den Knochenzement im Schlauch oder Schnorchel (40) vorwärtsbewegt, so daß im entfalteten Zustand der mit dem applizierten Knochenzement gefüllte Schlauch oder Schnorchel (40) die Markhöhle im wesentlichen ganz ausfüllt.

2. Applikationssystem nach Anspruch 1, welches ein- oder mehrteilig, vorzugsweise zweiteilig ausgebildet ist.

3. Applikationssystem nach Anspruche 1 oder 2, wobei das Auslaßstück derart ausgebildet ist, daS es ohne Absatz entlang der inneren Oberflächen an die Auslaßöffnung des Behältnisses angepaßt ist, und wobei das Auslaßstück vorzugweise einen trichterförmigen Teil aufweist.

4. Applikationssystem nach einem der Ansprüche 1 bis 3, wobei der faltbare Schlauch oder Schnorchel im entfalteten Zustand etwa 3 bis 25 cm, vorzugsweise 5 bis 20 cm lang ist, und wobei der faltbare Schnorchel oder Schlauch vorzugsweise so ausgebildet ist, daß er leicht in die durch das Applikationssystems zu befüllende Markhöle einführbar ist und nach dem Befüllen der Markhöhle zurückgezogen werden kann.

5. Applikationssystem nach einem der Ansprüche 1 bis 4, welches eine ein- oder mehrteilige Kappe aufweist, die derart auf das Behältnis aufsetzbar ist, beispielsweise mit einem Bajonettverschluß oder Schraubverschluß, daß die inneren Oberflächen der Kappe und des Behältnisses ohne Absatz ineinander übergehen, wobei die Kappe vorzugsweise derart ausgebildet ist, daß sie auch eine nicht-rotationssymmetrische Auslaßöffnung, beispielsweise eine quere Auslaßöffnung des Behältnisses, absatzfrei verlängern kann, und/oder wobei die Einstellung der Kappe in ihrer Position bezüglich des Behältnisses reproduzierbar ist, beispielsweise durch Anschlag des Bajonetts oder Abstimmung der Gewinde des Schraubverschlusses.

6. Applikationssystem nach Anspruch 5, wobei die Kappe zweiteilig ausgebildet ist und den faltbaren Schnorchel oder Schlauch aufweist, und wobei der innere Durchmesser von Kappenhauptteil und Schnorchel bzw. Schlauch an der Übergangsstelle im wesentlichen identisch ist.

7. Applikationssystem nach einem der Ansprüche 1 bis 6, wobei der Schnorchel oder Schlauch in der Weise aufgezogen ist, daß er durch mindestens ein scharfkantiges Außenprofil mit Hinterschneidung an der Kappe festgehalten und am Zurückgleiten gehindert wird, und/oder wobei der faltbare Schnorchel oder Schlauch mittels eines Gewindes oder einer Schlauchklemme am Auslaßstück befestigt und gesichert ist.

8. Applikationssystem nach einem der Ansprüche 1 bis 7, wobei der Schnorchel oder Schlauch sternförmig oder in anderer Weise faltbar ist, wobei der faltbare Schnorchel oder Schlauch vorzugsweise an seinem freien Ende leichter entfaltbar ist als an seinem an oder in der Nähe des Auslaßstückes gelegenen anderen Ende.

9. Applikationssystem nach einem der Ansprüche 1 bis 8, wobei als Material ein sterilisierbarer Kunststoff, z.B. TPX, ein Gummi oder Silikon, Polycarbonat oder ein ähnliches Material, ein Metall oder Glas verwendet wird, und/oder wobei als Material für den faltbaren Schnorchel oder Schlauch ein glattes elastisches Material oder ein Verbundwerkstoff verwendet wird, welcher windkesselfunktion ausüben kann, z.B. Polytetrafluoräthylen.

10. Applikationssystem nach einem der Ansprüche 1 bis 9, wobei der Knochenzement ein PMMA-Knochenzement oder eine anorganische Zementmasse ist.

## Claims

1. An application system for application of bone cement from a receptacle (5) into a bone cavity, including an outlet part (16) being adapted to said receptacle and with an elongated application part, characterized in that there is no shoulder along the inner surface of the components of said application system, and in that said application part being a one-piece collapsible tube or snorkel (4) which is attached to the outside of said outlet part (16) and wherein its length and material are chosen so that said tube or snorkel (4) deforms and expands through said bone cement being input during application and moves forward during subsequent contraction so that said tube or snorkel (40) filled with said applied bone cement is substantially filling said bone cavity in the unfolded condition.

2. The application system of claim 1 which is formed in more or more parts, preferably in two parts.

3. The application system of claim 1 or 2, wherein said outlet part being formed so that it is adapted to the outlet opening of said receptacle along the inner surfaces and wherein said outlet part preferably including a funnel-shaped portion.

4. The application system of claims 1 to 3, wherein the length of said collapsible tube or snorkel in its unfolded condition is about 3 to 25 cm, preferably 5 to 20 cm, and wherein said collapsible snorkel or tube is preferably formed so that it can easily be inserted into the bone cavity to be filled by said application system and is adapted to be removed after filling said bone cavity.

5. The application system of claims 1 to 4, including a cap with one or more parts, which can be put on said receptacle for example by a bayonet joint or a screw closure so that the inner surfaces of said cap and said receptacle fit together without a shoulder, wherein said cap is preferably formed so that it is also adapted to extend a not dynamically balanced outlet opening without shoulder, for example a transversal outlet opening of said receptacle, and/or wherein the adjustment of the position of said cap is reproducible with regard to said receptacle for example by joining the bayonet or adapting the threads of said screw closure.

6. The application system of claim 5, wherein said cap is formed in two parts and includes said collapsible snorkel or tube, and wherein the inner diameter of the main part of said cap and of said snorkel or tube is substantially the same at the junction.

7. The application system of claims 1 to 6, wherein said snorkel or tube is attached so that it is held at said cap by at least one sharp outer profile being undercut and prevented from sliding back, and/or wherein said collapsible snorkel or tube is attached and secured by means of a thread or a tube clamp at said outlet part.

8. The application system of claims 1 to 7, wherein said snorkel or tube may be folded star-shaped or otherwise, wherein said collapsible snorkel or tube is preferably easier to unfold at its free end in comparison to other end being at or close to said outlet part.

9. The application system of claims 1 to 8, wherein the material is chosen from a sterilisable plastic, e.g. TPX, rubber or silicone, polycarbonate or a similar material, metal or glass, and/or wherein the collapsible snorkel or tube can be made of a smooth, resilient material or a composite which can perform a windkessel-function, e.g. polytetrafluoroethylene.

10. The application system of claims 1 to 9, wherein said bone cement includes polymethylmethacrylate bone cement or an anorganic cement mass.

## Revendications

1. Système d'application pour appliquer le cément osseux à partir d'un récipient (5) dans un canal médullaire à l'aide d'un élément d'évacuation (16) adapté au récipient et d'une pièce allongée servant à l'application, caractérisé par le fait qu'aucune saillie n'est présente sur la surface interne des pièces du système d'application et que la pièce pour application est un tuyau souple ou un tube (4) fait d'une seule pièce, pliable, fixé sur l'extérieur de la pièce pour application (16) et dont la longueur et la matière sont choisis de sorte à ce que le tuyau flexible ou le tube se déforme et se gonfle pendant l'application sous l'effet du cément osseux introduit et que la contraction qui s'ensuit fait progresser le cément osseux dans le tuyau flexible ou dans le tube (4), si bien qu'à l'état déplié le tuyau flexible ou le tube (40) renfermant le cément osseux appliqué occupe presque entièrement le canal médullaire.

2. Système d'application selon revendication 1, réalisé en une ou en plusieurs parties, de préférence en deux parties.

3. Système d'application selon revendication 1 ou 2, la pièce d'évacuation étant réalisée de telle façon qu'elle s'adapte à l'ouverture du récipient sans faire aucune saillie le long des surfaces internes, l'élément d'évacuation se présentant de préférence comme une pièce en forme d'entonnoir.

4. Système d'application selon l'une des revendications 1 à 3, le tuyau flexible ou le tube ayant une longueur de quelque 3 à 25 cm à l'état déplié, de préférence de 5 à 20 cm de long, le tube ou le tuyau flexible pliable étant réalisé de préférence de sorte à pouvoir être facilement introduit à travers le système d'application dans le canal médullaire à remplir et qu'il puisse être retiré après remplissage du canal médullaire.

5. Système d'application selon l'une des revendications 1 à 4 qui présente une calotte constituée d'une ou de plusieurs parties, qui peut être installée sur le récipient, par exemple à l'aide d'une fermeture à baïonnette ou d'une fermeture à vis, de telle sorte que les surfaces internes de la calotte et du récipient passent de l'une à l'autre sans former de saillie, la calotte étant réalisée de préférence de sorte à pouvoir également prolonger une ouverture d'évacuation non symétrique à la rotation sans former de saillie, par exemple une ouverture d'évacuation transversale du récipient, et/ou l'ajustement de la calotte dans sa position par rapport au récipient étant reproductible, par exemple au moyen de la butée de la baïonnette ou par accord du filetage de la fermeture à vis.

6. Système d'application selon revendication 5, la calotte étant réalisée en deux parties et présentant le tube ou le tuyau flexible pliable, et le diamètre interne de la partie principale de la calotte et du tube ou du tuyau flexible étant essentiellement identique à la partie constituant la transition.

7. Système d'application selon l'une des revendications 1 à 6, le tube ou le tuyau flexible étant tendu de manière à être maintenu contre la calotte par au moins un profil extérieur à bord acéré avec contre-dépouille, et qui l'empêche de glisser en arrière, et/ou le tube ou le tuyau flexible pliable étant fixé par un filetage ou une pince à tuyau contre l'élément d'évacuation et bloqué.

8. Système d'application selon l'une des revendications 1 à 7, le tube ou le tuyau flexible étant pliable en forme d'étoile ou d'une autre manière, le tube ou le tuyau flexible pliable étant de préférence plus facilement dépliable à son - extrémité libre qu'à son autre extrémité située contre l'élément d'évacuation ou à proximité de lui.

9. Système d'application selon l'une des revendications 1 à 8, la matière utilisée étant une matière plastique stérilisable, par exemple du TPX, du caoutchouc ou des silicones, du polycarbonate ou une autre matière semblable, un métal ou du verre, et/ou la matière du tube ou du tuyau flexible étant une matière lisse et élastique ou un matériau composite pouvant exercer une fonction de réservoir d'air, du polytétrafluoro-éthylène, par exemple.

10. Système d'application selon l'une des revendications 1 à 9, le cément osseux étant un cément en matière plastique au polymétacrylate de méthyle ou une masse de cément inorganique.
